# EUROPEAN PATENT APPLICATION

(11) **EP 1 452 185 A1**
(43) Date of publication of application: **01.09.2004**
(21) Application number: 03100508.5
(22) Date of filing: 28.02.2003
(51) Int. Cl.: A61K 41/00

(54) **Activation and production of radiolabeled particles**

(71) Applicant: EURATOM, 1049 Brussels (BE)
(72) Inventor: Magill, Joseph, 76139, KARLSRUHE (DE); Galy, Jean, 76131, KARLSRUHE (DE); Apostolidis, Christos, 69119, HEIDELBERG (DE); Somers, Joseph, 76227, KARLSRUHE (DE); Jehenson, Philippe, 75014 Paris (FR)
(74) Representative: Kihn, Pierre Emile Joseph

(57) **Abstract**

A method for activating particles for internal radiopharmaceutical use, said particles comprising precursor nuclides to be activated, is disclosed. The method comprises:
directing a high-intensity laser beam onto converting means to produce an irradiating field; and
irradiating said particles comprising precursor nuclides in said irradiating field to activate said precursor nuclides, thereby obtaining radiolabeled particles.

## Description

### FIELD OF THE INVENTION

The present invention generally relates to the activation and production of radiolabeled particles for internal radiopharmaceutical use.

### BACKGROUND OF THE INVENTION

Radiolabeled particles are currently used for two main radiopharmaceutical applications: internal radiation therapy and nuclear medical imaging.

As is well known, such radiolabeled particles generally consist of radion u-clides combined with carrier materials. Their dimensions vary from a few nanometers to several hundreds of micrometers and the particles are generally of spherical form. Therefore, they are often referred to as microparticles, microspheres, nanoparticles, beads or microcapsules.

Many different types of nano- and microparticles have been developed. The carrier material may be based on polymers, polymeric resins, albumin, or inorganic materials such as e.g. glass. The choice of radionuclide depends on the intended use of the radiolabeled particle. For radiotherapy, the radionuclide must have an appropriate radiation spectrum for treating small to large multiple tumours. Generally, β- emitters are used. For nuclear medical imaging, the radionuclide should be a β+ emitter, so that γ-rays produced by the annihilation process can be detected for imaging purposes

The preparation of radiolabeled micro- and nanoparticles has been widely described in the scientific and patent literature. In most methods, particles comprising stable precursor nuclides combined with carrier material are prepared, and the particles are subsequently activated in a nuclear reactor by neutron bombardment. In some methods, the radionuclides are first produced, and particles are then formed by combining the radionuclides with the carrier material.

A disadvantage of these methods is that they require a nuclear reactor for activating the precursor nuclide into the radioisotope suitable for radiotherapy or imaging.

### OBJECT OF THE INVENTION

The object of the present invention is to provide an alternative way of preparing radiolabeled particles for radiopharmaceutical use. This object is achieved by a method as claimed in claim 1.

### SUMMARY OF THE INVENTION

The present invention proposes a method for activating particles for internal radiopharmaceutical use, the particles comprising precursor nuclides to be activated. According to the invention, the method comprises:
directing a high-intensity laser beam onto converting means to produce an irradiating field; and
irradiating the particles comprising precursor nuclides in the irradiating field to activate the precursor nuclides, thereby obtaining radiolabeled particles.

It will be appreciated that the present method uses a laser to produce the irradiating field that will induce the nuclear reactions required to activate the precursor nuclides. This eliminates the need for a nuclear reactor. The interaction of the high-intensity laser beam with the converting means produces a high-energy irradiating field of photons or protons, depending on the laser intensity and the converting means. The use of a laser beam for activating particles such as microspheres and nanoparticles instead of a nuclear reactor proves extremely advantageous, in terms of cost, size, operation and maintenance. So-called tabletop-lasers are very compact and are particularly suitable for installation in hospitals. The present method can thus easily be implemented in hospitals or other radiotherapy treatment centers, without relying on distant nuclear reactors.

In a preferred embodiment, the irradiating field is a bremsstrahlung photon field. Therefore, the converting means preferably includes a solid target, such as e.g. a metallic foil. More preferably, the converting means includes a first target part on which said laser beam is focused, thereby creating high-energy electrons. A second target part is placed behind the first target and the high-energy electrons impinge thereon, so that it acts as a bremsstrahlung converter creating high-energy photons. The production of photons thus permits the induction of photonuclear reactions (noted (γ, n)) in the particles. Absorption of high-energy electromagnetic radiation in the form of gamma-ray photons-produced by the interaction of the laser beam with the converting means-causes a precursor nuclide to eject a neutron, resulting in the formation of a radioactive isotope of the same element.

Preferred materials for the metallic target, respectively first and second target parts, are tantalum, tungsten, platinum or copper.

The present method allows activation of any particles suitable for radiopharmaceutical use, which comprise precursor nuclides that can be activated through (γ, n) reactions. In particular, the method allows activation of particles comprising as precursor nuclides a stable isotope of an element from the following list: Ag, Au, Br, C, Cd, Ce, Cl, Cr, Cu, Er, Eu, F, Fe, Ga, Gd, Ge, I, In, Ir, K, Kr, Lu, Mo, N, Nd, Ni, O, Os, P, Pd, Pr, Pt, Rb, Re, Ru, Sb, Sc, Se, Sm, Sn, Te, Ti, W, Xe, Yb, Zn. Irradiating stable isotopes of these elements with photons will thus produce radionuclides having radiation spectra appropriate for medical use.

For cancer therapy, particles labelled with a β- emitting radionuclide are preferred. A preferred list of suitable radionuclides is the following: ⁷⁰Ga, ⁷⁵Ge, ⁸⁰Br, ⁸¹Se, ^{81m}Se, ^{85m}Kr, ⁸⁶Rb, ⁹⁹Mo, ¹⁰³Ru, ¹⁰⁸Ag, ¹⁰⁹Pd, ^{109m}Pd, ^{114m}In, ¹¹⁵Cd, ¹²¹Sn, ¹²²Sb, ¹²⁷Te, ¹²⁹Te, ¹³³Xe, ¹³⁵Xe, ¹⁴¹Ce, ¹⁴⁷Nd, ¹⁴⁹Nd, ¹⁵³Sm, ^{152m}Eu, ¹⁵⁹Gd, ¹⁶⁹Er, ¹⁷⁵Yb, ^{176m}Lu, ¹⁸⁵W, ¹⁸⁶Re, ¹⁹¹Os, ¹⁹²Ir, ¹⁹⁷Pt, ^{197m}Pt. Hence, to produce particles labelled with the preceding radionuclides through (γ, n) reactions with the present method, the particles to be irradiated should normally comprise as precursor nuclides a stable isotope of the same element as the desired radionuclide, with a mass number superior by one unit to the mass number of the desired radionuclide.

For nuclear medical imaging, particles labelled with positron (β+) emitters are preferred. The β+ emitting nuclides interact with electrons via an annihilation process, which results in the production of two 0.511 MeV photons that can be detected e.g. by positron emission tomography cameras. A preferred list of suitable radionuclides is the following: ¹¹C, ¹³ N, ¹⁵O, ¹⁸F , ³⁰P, ^{34m}Cl, ³⁸K, ⁴⁴Sc, ⁴⁵Ti , ⁴⁹Cr, ⁵³Fe, ⁵⁷Ni, ⁶²CU, ⁶⁴CU, ⁶³Zn, ⁶⁸Ga, ⁶⁹Ge, ⁷⁸Br, ⁸⁴Rb, ⁹⁵Ru, ¹⁰¹Pd, ¹⁰⁶Ag, ¹⁰⁵Cd, ¹¹²In, ¹²⁰Sb, ¹²⁶I, ¹⁴⁰Pr, ¹⁴¹Nd, ¹⁹⁰ⁿIr, ¹⁹⁶Au. Again, to produce particles labelled with the preceding radionuclides through (γ, n) reactions with the present method, the particles to be irradiated should comprise as precursor nuclide a stable isotope of the same element as the desired radionuclide, with a mass number superior by one unit to the mass number of the desired radionuclide.

Hence, the present method allows production of both β- and β+ emitting radiolabeled particles. As a matter of fact, when preparing particles from a given precursor element and carrier material, the precursor element will often include different naturally occurring stable isotopes of this element. As a result, upon irradiation in the photon irradiating field, the radiolabeled particles will emit both β- and β+ radiations. Hence, the present method allows producing radiolabeled particles that can simultaneously be used for therapeutical and imaging purposes, thereby allowing the treatment of tumours and observation of the effect. This was not possible with conventional activation methods, since neutron bombardment cannot lead to the production of both a β- emitter and a β+ emitter (main decay). In this connection, the following elements are preferred as precursors: Ga, Zn, Ge, Br, Kr, Mo, Pd, Cd, Sb, Nd, Er.

In another embodiment of the present method, the irradiating field is a proton field. Therefore, the converting means preferably is a target of carbon and hydrogen containing material. The production of protons thus allows to induce nuclear reactions involving absorption of protons by the precursor nuclides with emission of neutrons (noted (p ,xn)).

The intensity of the laser beam used to produce the irradiating field by interaction with the converting means needs to be of sufficient energy so that the photons, respectively the protons, produced are of sufficient energy to drive the (γ, n) reactions, respectively the (p, xn) reactions. Preferably, the intensity of the laser is of at least 10¹⁹ W/cm², more preferably about 10²⁰ W/cm².

During irradiation, the particles are advantageously placed in a container. The container is preferably made of a light element (e.g. aluminium) to limit absorption of the produced radiations by the container itself.

The distribution of the particles in the zones to be treated or analysed depends on the size of the particles. A preferred size range for the particles is between 10 nm and 500 µm, more preferably between 1 and 100 µm, depending on the application. The particles may be of various shapes, although spherical particles are generally preferred.

According to another aspect of the present invention, a method for producing radiolabeled particles for internal radiopharmaceutical use comprises a first step of providing particles comprising precursor nuclides and a subsequent step of activating the particles to obtain radiolabeled particles. The activation step comprises:
directing a high-intensity laser beam onto converting means to produce an irradiating field; and
irradiating the particles comprising precursor nuclides in the irradiating field.

In the present method, nuclear reactions required for activation of the particles are induced by the laser-produced irradiating field, thereby eliminating the need for a nuclear reactor. This method avoids one particularly constraining aspect of conventional methods, where radiolabeled particles must be activated in nuclear reactors out of hospitals. Indeed, with the present method, the important activation step of the production of radiolabeled particles can be carried out on the site of use, since today's laser systems are well suited to be installed in hospitals. This has an important impact on the types of radiolabeled particles that can be produced, since it allows production of radiolabeled particles comprising radionuclides having a short half-life time, and which could not be used before in hospitals far away from nuclear reactors.

The method advantageously includes a further step of suspending the radiolabeled particles in an appropriate aqueous media to be injected into a patient's body, e.g. in a tumour and/or in a zone to be analysed.

When producing the particles to be irradiated in a photon field, the precursor nuclides are selected in function of the desired application, as already explained. For cancer therapy, the precursor nuclides used for preparing the particles are preferably stable isotopes of an element that will be converted, through (γ, n) reactions, into beta- emitting radionuclides. For medical nuclear imaging, the precursor nuclides used for preparing the particles are preferably stable isotopes of an element that will be converted, through (γ, n) reactions, into β+ emitting radionuclides. In addition, the particles may comprise as precursor nuclides a mixture of stables isotopes, part of the isotopes being activated into β- emitting radionuclides and part of the isotopes being activated into β+ emitting radionuclides.

### BRIEF DESCRIPTION OF THE DRAWING

The present invention will now be described, by way of example, with reference to the accompanying drawing, in which:

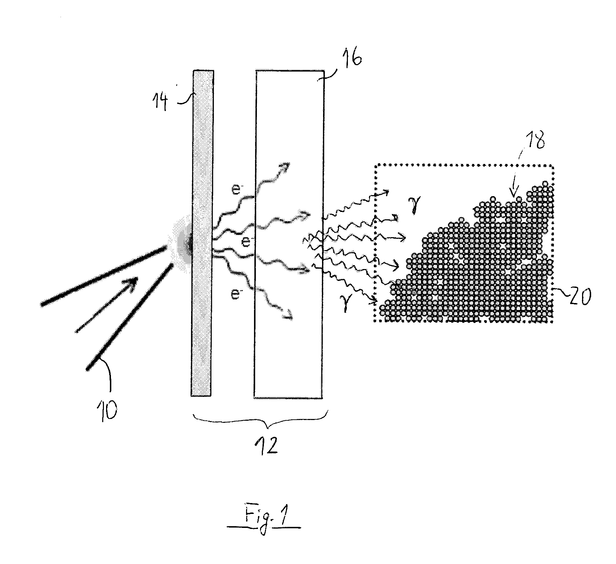

FIG. 1: is a sketch of the experimental set-up used to implement a preferred embodiment of the present method.

### DETAILED DESCRIPTION OF A PREFERRED EMBODIMENT

A preferred embodiment of a method according to the invention will now be described in more detail, wherein radiolabeled particles are produced using an activation step involving photon irradiation. The present method comprises two main steps of: (1) preparing particles comprising precursor nuclides, and (2) irradiating the particles in a laser-produced photon irradiating field to activate them into radiolabeled particles.

### I. Preparation of the particles

Particles for pharmaceutical use are known as microparticles, nanoparticles, microspheres, microcapsules or beads. They are typically of spherical shape and have dimensions (mean diameters) ranging between 10 nm and 500 µm.

For internal patient use, the particles should preferably be in a form that is insoluble in *"in vivo"* conditions, i.e. in cellular media. If particles comprising stable nuclides can be elaborated into particles of a chemical form that is insoluble in cellular media, then there is no need for carrier material. For example, a carrier is not required for insoluble oxides of the lanthanides and of other metals predominantly on the left side of the transition metal block of the periodic table.

However, in many cases the stable radionuclides are combined with carrier material to form particles. In particular, coating and encapsulation with the carrier material can avoid the necessity for insoluble precursor nuclide compounds. Moreover, a carrier material is preferably used for dilution of the radionuclide or density adjustment (particularly to that of the cellular media). The carrier material may be based on polymers, polymeric resins, albumin, or inorganic materials such as e.g. glass. Frequently, microspheres are prepared by producing spheres of the carrier material and combining the radionuclide with the sphere of carrier material e.g. by complexation.

Particle coatings can also be considered for soluble (or even insoluble) materials, whereby the coating should be insoluble and preferably have a thickness between 1 and 100 µm, more preferably below 20 µm.

It will be understood that since a given metal may naturally occur under two or more stable isotopic forms, a particle prepared from this metal (without prior isotopic separation), to comprise as precursor nuclide one of these stable isotopes, may also comprise other stable isotopes of this metal.

A variety of methods have been proposed for producing microspheres and nanoparticles, and will not be discussed in detail herein. Any method allowing to produce particles comprising precursor nuclides optionally combined with carrier material can be used for the preparation of particles for the production of radiolabeled particles according to the present method.

Hence, the term "particles comprising precursor nuclides" herein refers to any particles comprising precursor nuclides, and in particular to: particles of a precursor nuclide in pure or compound form that is insoluble in cellular media; particles consisting of precursor nuclides encapsulated in carrier material; or particles of carrier material to which precursor nuclides are bound.

### II. Activation of the particles

According to the present method, after production of the particles comprising the precursor nuclides, the latter are activated by bremsstrahlung photons generated by interaction of a laser beam onto a metallic target.

A preferred experimental set-up for conducting the irradiating step is shown in Fig.1. A high-intensity laser beam 10 is focused onto a converting means generally indicated 12. The converting means 12 itself preferably comprises two target parts, more specifically of two sheets 14, resp. 16, of tantalum with a thickness of 50 µm and 1 mm, respectively. The laser beam impinges onto the first target part 14. The angle of incidence of the laser beam 10 is preferably less than 45° in parallel polarization, as this geometry allows for high absorption of laser light into a plasma. The second target part 16 is placed behind the first target part 14 (with regard to the incident laser beam). The particles comprising the precursor nuclides 18 to be activated are placed in bulk in a container 20, preferably made of aluminium, which is positioned behind the second part 16.

The incident laser beam 10 is preferably generated by means of a so-called tabletop laser. Preferred laser parameters are the following:
Laser pulse energy: 1 J (1 Joule)
Laser pulse length: 50x10⁻¹⁵ s (50 femtoseconds)
Laser intensity: 10²⁰ W/cm²
Focal spot diameter: 5 µm²

The high-intensity laser beam 10 produces a relativistic plasma on the surface of the first target part 14. Plasma electrons are accelerated to relativistic energies within the intense laser field. These fast electrons impinge on the second part 16, which serves as an efficient bremsstrahlung converter. As a result, an irradiating field of high-energy bremsstrahlung photons is produced behind the second part 16 (schematically illustrated by γ waves in Fig.1), whereby the particles 18 are irradiated with these bremsstrahlung photons. With these laser operating parameters, photons having an energy of up to 30 MeV and more can be obtained.

The photons impinging onto the particles induce (γ, n) photonuclear reaction in the precursor nuclides. Indeed, absorption of high-energy electromagnetic radiation in the form of gamma-ray photons causes the nucleus of a precursor nuclide to eject a neutron, resulting in the formation of a radioactive isotope. As a result, radiolabeled particles are obtained.

Regarding more particularly the laser beam parameters, the laser pulse repetition rate should preferably be as high as possible, for productivity reasons. Presently laser technology already permits these goals, but, with the actual developments of the laser technology, it should soon be possible to implement the present method with lasers system having a pulse repetition rate of higher than 10 Hz, higher energy per shot and likely higher intensity.

### III. Choice of the radionuclide

The choice of radionuclide depends on the intended use of the radiolabeled particle. For radiotherapy, the radionuclide must have an appropriate radiation spectrum for treating small to large multiple tumours. Generally, β-emitters are used. For nuclear medical imaging, the radionuclide should be a β+emitter, so that γ-rays are produced due to annihilation process.

A list of preferred β- emitting nuclides which can be produced by (γ, n) reactions and that are of interest for cancer therapy is given in table 1. These nuclides (second column) can be obtained by irradiating particles comprising as precursor nuclides a stable isotope indicated in the first column (the precursor isotope is underlined; the abundance percentage of this isotope is also indicated in brackets). Half-life, Main β- rays and main γ-rays are also indicated, with their respective emission probability.

**Table 1.**

| *List of β- emitters* | | | | |
|---|---|---|---|---|
| **Stable isotope precur- sor(s)** | **Nuclide** | **Half-life** | **Main β-(MeV)** | **Main γ** |
| ^{69,}^{**71**}**Ga** (40%) | ⁷⁰**Ga** | 21.14 min | 1.7 (99%) | 1.04 MeV (0.6%) |
| ^{70,72-74,}^{**76**}**Ge** (7%) | ⁷⁵**Ge** | 1.38 h | 1.2 (87%) | 265 keV (13%) |
| ^{79,}^{**81**}**Br** (49%)) | ⁸⁰**Br** | 18 min | 2 (84%) | 612 keV (7%) |
| ^{74,76-78,80,}^{**82**}**Se** (9%) | ⁸¹**Se** | 18.45 min | 1.6 (98%) | 276 keV (0.8%) |
| | ^{81m}**Se** | 57.8 min | 1.2 (0.06%) | 103 keV (13%) |
| ^{78,80,82-84,}^{**86**}**Kr** (17%) | ^{85m}**Kr** | 4.4 h | 0.84 (79%) | 151 keV (75%) |
| ^{85,}^{**87**}**Rb**(29%) | ⁸⁶**Rb** | 18.6d | 1.7(91%) | 1.1 MeV (8%) |
| ^{94-98,}^{**100**}**Mo** (10%) | ⁹⁹**Mo** | 2.75 d | 1.2 (82%) | 739 keV (12%) |
| ^{98-102,}^{**104**}**Ru** (19%) | ¹⁰³**Ru** | 39.26 d | 0.225 (90%) | 497 keV (89%) |
| ^{107,109}Ag (48%) | ¹⁰⁸**Ag** | 2.37 min | 1.6 (96%) | 633 keV (0.2%) |
| ^{102,104-106,108,}^{**110**}_{**Pd**} (12%) | ¹⁰⁹**Pd** | 13.7 d | 1.03 (99%) | 311 keV (0.03%) |
| | ^{109m}**Pd** | 4.7 min | - | 189 keV (48%) |
| ^{113,}^{**115**}In (96%) | ^{114m}**In** | 49.51 d | 0.35 (4%) | 190 keV (15%) |
| ^{106,108,110-114,}^{**116**}**Cd** (7%) | ¹¹⁵**Cd** | 2.23 d | 1.1 (63%) | 527 keV (27%) |
| ^{112,114-119,}^{**122**}^{,124}**Sn** (5%) | ¹²¹**Sn** | 1.13 d | 0.383 (100%) | - |
| ^{121,}^{**123**}**Sb** (43%) | ¹²²**Sb** | 2.72 d | 1.4 (66%) | 564 keV (70%) |
| ^{120,122-126,}^{**128**}^{,130}**Te** (32%) | ¹²⁷**Te** | 9.35 h | 0.694 (99%) | 498 keV (1 %) |
| | ¹²⁹**Te** | 69 min | 1.5 (89%) | 459 (7%) |
| ^{124,126,128-132,}^{**134**}^{,136}**Xe** 10%) | ¹³³**Xe** | 5.24 d | 0.346 (99%) | 81 keV (37%) |
| | ¹³⁵**Xe** | 9.14 h | 0.908 (95%) | 250 keV (90%) |
| ^{136,138,140,}^{**142**}**Ce** (11%) | ¹⁴¹**Ce** | 32.5 d | 0.436 (70%) | 145 keV (48%) |
| ^{142-146,}^{**148**}^{,}^{**150**}**Nd** (5%) | ¹⁴⁷**Nd** | 10.98 d | 0.804 (81 %) | 531 keV (13%) |
| | ¹⁴⁹**Nd** | 1.73 h | 1.5 (28%) | 211 keV (25%) |
| ^{144,147-150,}^{**154**}**Sm** (23%) | ¹⁵³**Sm** | 1.93 d | 0.713 (43%) | 103 keV (28%) |
| ^{151,}^{**153**}_{**Eu**} (53%) | ^{152m}**Eu** | 9.31 h | 1.9 (68 %) | 841 keV (15%) |
| ^{152,154-158,}^{**160**}**Gd** (22%) | ¹⁵⁹**Gd** | 18.5 h | 0.974 (62%) | 363 keV (10%) |
| ^{162,164,166-168,}^{**170**}**Er** (15%) | ¹⁶⁹**Er** | 9.4 d | 0.352 (58%) | - |
| ^{168,170-174,}^{**176**}**Yb** (13%) | ¹⁷⁵**Yb** | 4.2 d | 0.467 (87%) | 396 keV (7%) |
| ^{175,}^{**176**}**Lu** (3%) | ^{176m}**Lu** | 3.74 h | 1.2 (60%) | 88 keV (9%) |
| ^{180,182-184,}^{**186**}**W** (28%) | ¹⁸⁵**W** | 75 d | 0.432 (99%) | 125 keV(0.02%) |
| ^{185,}^{**187**}**Re** (63%) | ¹⁸⁶**Re** | 3.72 d | 1.1 (74%) | 137 keV (8%) |
| ^{184,186-190,}^{**192**}**Os** (41%) | ¹⁹¹**OS** | 15.4 d | 0.141 (100%) | 129 keV (29%) |
| ^{191,}^{**193**}**Ir** (63%) | ¹⁹²**Ir** | 73.83 d | 0.672 (48%) | 316 keV (83%) |
| ^{190,192,194-196,}^{**198**}**Pt** (7%) | ¹⁹⁷**Pt** | 19.89 h | 0.641 (81%) | 77 keV (17%) |
| | ^{197m}**Pt** | 1.97 h | 0.709 (3%) | 346 keV (11 %) |

A list of preferred β+ emitting nuclides which can be produced by (γ, n) reactions and that are of interest for medical nuclear imaging is given in table 2. These nuclides (second column) can be obtained by irradiating particles comprising as precursor nuclides a stable isotope indicated in the first column (the abundance percentage of this isotope is also indicated in brackets). Half-life, Main β+ rays (emission probability indicated in brackets) and threshold for the (γ, n) reaction are also indicated.

**Table 2.**

| *List of positron (β+) emitters* | | | | |
|---|---|---|---|---|
| **Stable iso- tope(s) precur- sor** | **Nuclides** | **Half-life** | **Main β+ (MeV)** | **Threshold for (γ,n) reaction (MeV)** |
| ¹² C (99%) | ¹¹C | 20.4 min | 1.98(100%) | 18 |
| ¹⁴ N (99%) | ¹³N | 10 min | 2.22 (100%) | 15 |
| ¹⁶O(99%) | ¹⁵O | 2 min | 2.75 (100%) | 17 |
| ¹⁹F (100%) | ¹⁸F | 1.8 h | 1.65(100%) | 10 |
| ³¹P (100%) | ³⁰P | 2.5 min | 4.2(100%) | 12.3 |
| ³⁵Cl (76%) | ^{34m}Cl | 32 min | 3.5 (28%) | 12.6 |
| | | | 2.3 (24%) | |
| ³⁹K (93%) | ³⁸K | 7.64 min | 3.7 (99%) | 13.1 |
| ⁴⁵Sc (100%) | ⁴⁴Sc | 3.93 h | 2.5 (99%) | 11.3 |
| ⁴⁶Ti (8%) | ⁴⁵Ti | 3.1 h | 2.1(100%) | 13.2 |
| ⁵⁰Cr (4%) | ⁴⁹Cr | 42 min | 2.47 (52%) 2.54 (37%) | 13.0 |
| ⁵⁴Fe (6%) | ⁵³Fe | 8.5 m | 3.74 (58%) 3.37 (39%) | 13.4 |
| ⁵⁸Ni (68%) | ⁵⁷Ni | 1.5 d | 1.87 (34%) | 12.2 |
| ⁶³CU (69%) | ⁶²Cu | 9.7 min | 3.9 (99%) | 10.9 |
| ⁶⁵Cu (31%) | ⁶⁴Cu | 12.7 h | 0.578 (40%) | 9.9 |
| ⁶⁴Zn (49%) | ⁶³Zn | 38.5 min | 3.37 (84%) | 11.9 |
| ⁶⁹Ga(60%) | ⁶⁸Ga | 1.1 h | 2.92 (97%) | |
| ⁷⁰Ge (21%) | ⁶⁹Ge | 1.6 d | 2.23 (31%) 1.12 (37%) | 11.5 |
| ⁷⁹Br (49%) | ⁷⁸Br | 6.46 min | 3.6 (86%) | |
| ⁸⁵Rb (72%) | ⁸⁴Rb | 33 d | 1.8 (68%) | 10.5 |
| ⁹⁶Ru (6%) | ⁹⁵Ru | 1.6 h | 2.26 (37%) 1.13(25%) | |
| ¹⁰²Pd(10%) | ¹⁰¹Pd | 8.5 h | 1.8 (57%) 1.2 (22%) | 10.6 |
| ¹⁰⁷Ag (52%) | ¹⁰⁶Ag | 24 min | 2.97 (83%) 2.45 (16%) | 9 6 |
| ¹⁰⁶Cd (1.2%) | ¹⁰⁵Cd | 55 min | 2.71 (54%) 1.10 (50%) | 10.6 |
| ¹¹³In(4%) | ¹¹²In | 15 min | | 9.4 |
| ¹²¹ Sb (57%) | ¹²⁰Sb | 15.9 min | 2.68 (98%) | 9.2 |
| ¹²⁷I (100) | ¹²⁶I | 13 d | 0.826 (32%) | 9.1 |
| ¹⁴¹Pr (100%) | ¹⁴⁰Pr | 3.4 m | 3.39 (99%) | 9.4 |
| ¹⁴²Nd (27%) | ¹⁴¹Nd | 2.5 h | 1.81 (98%) | 9.8 |
| ¹⁹¹Ir (37%) | ¹⁹⁰ⁿIr | 3.1 h | 0.470(94%) | |
| ¹⁹⁷Au (100%) | ¹⁹⁶Au | 6.18 d | 1.1 (67%) | 8.1 |

The present method thus permits the production of radiolabeled particles comprising a radionuclide listed in table 1 or 2. Such a radiolabeled particle is obtained by: (1) preparing a particle comprising a stable precursor nuclide indicated in the first column of the tables (generally having a mass number superior by one unit to the radionuclide); and (2) activating the particle in a photon irradiating field produced by interaction of a laser beam onto a solid target.

It will be noted that when irradiating two stable isotopes of a same element with photons, one isotope may be converted into a β- emitting radionuclide whereas the other isotope is converted into a β+ emitting radionuclide.

Hence, when the particles comprise a mixture of such precursor isotopes, the present method allows production of radiolabeled particles that simultaneously emit β+ and β- rays, so that they can be used for both cancer therapy and medical nuclear imaging (diagnosis or spatial localisation).

A list of elements that allow simultaneous production of β- and β+ emitters through (γ, n) reactions is given in table 3. The first column gives two precursor isotopes of a same element (abundance % in brackets), and the radionuclides produced by (γ, n) reactions are given in the second column. The half-lives of these radionuclides are indicated in the last column.

**Table 3.**

| *List of β+ and β- emitters that can simultaneously be produced from different isotopes* of a *same element with the present method.* | | |
|---|---|---|
| **Stable isotope(s) precursor** | **Radionuclides** | **Half-life** |
| ⁶⁹Ga (60%), | ⁶⁸Ga (β+), | 1.1 h |
| ⁷¹Ga (40%) | ⁷⁰Ga (β-) | 21 min |
| ⁶⁴Zn (49%), | ⁶³ Zn (β+), | 38 min |
| ⁷⁰Zn (0.6%) | ⁶⁹Zn (β-) | 56 min |
| ⁷⁰Ge (21 %), | ⁶⁹Ge (β+), | 1.6 d |
| ⁷⁶Ge (7%) | ⁷⁵Ge (β-) | 1.4 h |
| ⁷⁹Br (51 %), | ⁷⁸Br (β+), | 6 min |
| ⁸¹Br (49%) | ⁸⁰Br (β-) | 18 min |
| ⁸⁰Kr (2%), | ⁷⁹Kr (β+), | 1.5 d |
| ⁸⁶Kr (17%) | ^{85m}Kr (β-) | 4.5 h |
| ⁹²Mo (15%), | ⁹¹Mo (β+), | 15.5 m |
| ¹⁰⁰Mo (10%) | ⁹⁹mo (β-) | 2.75 d |
| ¹⁰²Pd (1%) | ¹⁰¹Pd (β+) | 8.5 h |
| ¹¹⁰Pd (13%) | ¹⁰⁹Pd (β-) | 13.7 h |
| ^{106,108}Cd (1%) | ^{105.107}Cd (β+) | 55 m, 6.5 h |
| ¹¹⁶Cd (7.5%) | ¹¹⁵Cd (β-) | 2.2 d |
| ¹²¹Sb (57%) | ¹²⁰Sb (β+) | 15.9 min |
| ¹²³Sb (43%) | ¹²²Sb (β-) | 2.7 d |
| ¹⁴²Nd (27%) | ¹⁴¹Nd (β+) | 2.5 h |
| ¹⁵⁰Nd (6%) | ¹⁴⁹Nd (β-) | 1.7 h |
| ¹⁶⁶Er (34%) | ¹⁶⁵Er (β+) | 10.4 h |
| ¹⁷⁰Er (15%) | ¹⁶⁹Er (β-) | 9.4 d |

The particle produced according to the present method may thus a combination of two different radionuclides (or elements); one radionuclide for producing an optimum β- radiation for therapy and the other one for producing the optimum β+ radiation for diagnosis or spatial location. This can be easily achieved by preparing the particles from the two appropriate kinds of precursor nuclides.

## Claims

1. A method for activating particles for internal radiopharmaceutical use, said particles comprising precursor nuclides to be activated, **characterised by**
directing a high-intensity laser beam onto converting means to produce an irradiating field; and
irradiating said particles comprising precursor nuclides in said irradiating field to activate said precursor nuclides, thereby obtaining radiolabeled particles.

2. The method according to claim 1, **characterised in that** said irradiating field is a bremsstrahlung photon field.

3. The method according to claim 2, **characterised in that** said converting means includes a metallic target.

4. The method according to claim 3, **characterised in that** said converting means comprises a first target part on which said laser beam is focused and a second target part behind the target that acts as a bremsstrahlung converter.

5. The method according to claim 3 or 4, **characterised in that** said metallic target, respectively target parts, are made of tantalum, tungsten, platinum or copper.

6. The method according to any one of claims 2 to 5, **characterised in that** each particle comprises as precursor nuclide a stable isotope selected of an element selected from the group comprising: Ag, Au, Br, C, Cd, Ce, Cl, Cr, Cu, Er, Eu, F, Fe, Ga, Gd, Ge, I, In, Ir, K, Kr, Lu, Mo, N, Nd, Ni, O, Os, P, Pd, Pr, Pt, Rb, Re, Ru, Sb, Sc, Se, Sm, Sn, Te, Ti, W, Xe, Yb, Zn.

7. The method according to any one of claims 2 to 6, **characterised in that** said activated radiolabeled particles comprise radionuclides selected from the group comprising: ⁷⁰Ga, ⁷⁵Ge, ⁸⁰Br, ⁸¹Se, ^{81m}Se, ^{85m}Kr, ⁸⁶Rb, ⁹⁹Mo, ¹⁰³Ru, ¹⁰⁸Ag, ¹⁰⁹Pd, ^{109m}Pd, ^{114m}In, ¹¹⁵Cd, ¹²¹Sn, ¹²²Sb, ¹²⁷Te, ¹²⁹Te, ¹³³Xe, ¹³⁵Xe, ¹⁴¹Ce, ¹⁴⁷Nd, ¹⁴⁹Nd, ¹⁵³Sm, ^{152m}Eu, ¹⁵⁹Gd, ¹⁶⁹Er, ¹⁷⁵Yb, ^{176m}Lu, ¹⁸⁵W, ¹⁸⁶Re, ¹⁹¹Os, ¹⁹²Ir, ¹⁹⁷Pt, ^{197m}Pt, ¹¹C, ¹³N, ¹⁵O, ¹⁸F, ³⁰P, ^{34m}Cl ³⁸K, ⁴⁴Sc, ⁴⁵Ti, ⁴⁹Cr, ⁵³Fe, ⁵⁷Ni, ⁶²CU, ⁶⁴CU, ⁶³Zn, ⁶⁸Ga, ⁶⁹Ge, ⁷⁸Br, ⁸⁴Rb, ⁹⁵Ru, ¹⁰¹Pd, ¹⁰⁶Ag, ¹⁰⁵Cd, ¹¹² In, ¹²⁰ Sb, ¹²⁶I, ¹⁴⁰Pr, ¹⁴¹Nd, ¹⁹⁰ⁿIr, ¹⁹⁶Au.

8. The method according to claim 1, **characterised in that** said irradiating field is a proton field.

9. The method according to claim 8, **characterised in that** said converting means comprises a solid target of carbon- and hydrogen-containing material.

10. The method according to any one of the preceding claims, **characterised in that** said laser beam impinging onto said converting means has an intensity of at least 10¹⁹ W/cm², preferably of about 10²⁰ W/cm² and above.

11. The method according to any one of the preceding claims, **characterised in that** said particles are in a chemical form that is insoluble in cellular media.

12. The method according to any one of the preceding claims, **characterised in that** each of said particles to be activated are particles of a stable isotope in a chemical form that is insoluble in cellular media.

13. The method according to any one of claims 1 to 11, **characterised in that** said particles comprising precursor nuclides are formed by combining precursor nuclides with carrier material.

14. The method according to any one of the preceding claims, **characterised in that** each of said particles comprises a stable precursor nuclide that will be activated into a β- emitting radionuclide as well as another stable precursor nuclide that will be activated into a β+ emitting radionuclide.

15. The method according to any one of the preceding claims, **characterised in that** the dimensions of said particles are in the range of 10 nm to 500 µm, preferably of 1 to 100 µm.

16. The method according to any one of the preceding claims, **characterised in that** said particles comprising precursor nuclides are placed in a container during irradiation.

17. The method according to any one of the preceding claims, **characterised in that** it is carried out on the site of use of said radiolabeled particles.

18. A method for producing radiolabeled particles for internal radiopharmaceutical use, comprising the steps of:
providing particles comprising precursor nuclides; and
activating said particles to obtain radiolabled particles;
**characterized in that** said activating step is carried out according to the method as claimed in any one of the preceding claims.

19. The method according to claim 18, **characterised by** the step of suspending the radiolabeled particles in an appropriate aqueous media for injection into a patient's body.
